Europäisches Patentamt

(19) European Patent Office

Office européen des brevets

(11) Numéro de publication: **0 143 809**

**B1**

(12) **FASCICULE DE BREVET EUROPEEN**

(45) Date de publication du fascicule du brevet: **18.01.89**

(51) Int. Cl.⁴: **C 12 N 5/00**

(21) Numéro de dépôt: **84901775.1**

(22) Date de dépôt: **27.04.84**

(86) Numéro de dépôt international:
**PCT/FR84/00118**

(87) Numéro de publication internationale:
**WO 84/04325 08.11.84 Gazette 84/26**

(54) **PROCEDE D'OBTENTION DE CULTURES D'HEPATOCYTES HUMAINS, LES CULTURES OBTENUES ET LEURS APPLICATIONS BIOLOGIQUES ET BIOCHIMIQUES.**

(30) Priorité: **29.04.83 FR 8307148**

(43) Date de publication de la demande:
**12.06.85 Bulletin 85/24**

(45) Mention de la délivrance du brevet:
**18.01.89 Bulletin 89/03**

(84) Etats contractants désignés:
**AT BE CH DE GB LI LU NL SE**

(56) Documents cités:
**FR-A-2 059 471**

**Chemical Abstracts, Vol. 98, No. 15, 11 April 1983 (Columbus, Ohio, US), C. Guguen-Guillouzo et al.: "Maintenance and reversibility of active albumin secretion by adult rat hepatocytes cocultured with another liver epithelial cell type", see page 433, abstract 123537m**

**Le dossier contient des informations techniques présentées postérieurement au dépôt de la demande et ne figurant pas dans le présent fascicule.**

(73) Titulaire: **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur: **GUILLOUZO, Christiane -**
**41, avenue du Mail**
**F-35000 Rennes (FR)**
Inventeur: **GUILLOUZO, André**
**41, avenue du Mail**
**F-35000 Rennes (FR)**
Inventeur: **BOUREL, Michel**
**4, rue de la Borderie**
**F-35000 Rennes (FR)**

(74) Mandataire: **Peaucelle, Chantal et al**
**S.C. Ernest Gutmann - Yves Plasseraud 67, boulevard Haussmann**
**F-75008 Paris (FR)**

# EP 0 143 809 B1

(56) Documents·cités:

**Chemical Abstracts, Vol. 98, No. 3, 17 January 1983 (Columbus, Ohio, US), G. Baffet et al.: "Hydrocortisone modulates the production of extra-cellular material and albumin in long-term cocultures of adult rat hepatocytes with other liver epithelial cells", see page 80, abstract 11721k**

**Experimental Cell Research, 143, pp 47-54, 1983**

**Biochem. and Bioph. Research Comm. Vol. 109, No. 2, p. 507-512, 1982**

# EP 0 143 809 B1

**Description**

L'invention a pour objet un procédé d'obtention de cultures d'hépatocytes humains, les cultures obtenues et leurs applications biologiques et biochimiques.

On sait que le foie constitue le siège de multiples fonctions. On conçoit donc l'intérêt de cultures d'hépatocytes humains. en particulier pour des études pharmacologiques concernant par exemple, la toxicité, la caractérisation des voies métaboliques d'une nouvelle molécule, ou en ou virologie pour l'étude par exemple de l'hépatite virale.

A cet égard, il est important de pouvoir disposer de populations homogènes.

De même, on mesurera l'intérêt de pouvoir disposer cultures de cellules normales ayant un caryotype normal.

Or, les travaux, peu nombreux, effectués à ce jour sur les cultures de foie humain ont été réalisées le plus souvent à partir de fragments tissulaires, provenant principalement de foies pathologiques ou de lignées d'hépatomes et conduisant à des populations hétérogènes.

Selon des techniques récentes, des hépatocytes viables ont été obtenus par perfusion enzymatique de fragments hépatiques provenant le plus souvent de foies subnormaux ou pathologiques.

Cependant, les hépatocytes mis en suspension ne peuvent survivre plus de quelques heures et ne permettent donc pas des études de longue durée. En culture, on a pu constater que les hépatocytes de petits mammifères ne conservent un phénotype stable que durant quelques jours au plus.

L'addition au milieu de culture de substances nutritives appropriées et l'utilisation de substrats organiques permettent de prolonger la survie mais sans le maintien, quantitativement, des fonctions spécifiques du foie.

Dans des travaux récents, les inventeurs ont obtenu une survie prolongée de cultures d'hépatocytes de rat, avec maintien de leur intégrité fonctionnelle pendant plusieurs semaines, en associant les hépatocytes de rat avec une cellule hépatique d'origine probablement biliaire, de même espèce.

L'état d'avancement des recherches des inventeurs dans ce domaine, qui ont par ailleurs mis au point un procédé d'isolement d'hépatocytes humains normaux en quantité importante, les a conduit à étudier de nouveaux moyens d'obtention de cultures d'hépatocytes humains répondant de manière plus satisfaisante aux exigences de la technique.

Il s'est ainsi avéré de manière surprenante que l'utilisation d'un environnement spécifique visà-àvis d'hépatocytes humains pouvait conférer à ces cultures d'hépatocytes des propriétés nettement supérieures à celles obtenues jusqu'alors.

L'invention a donc pour but de fournir un procédé de culture à long terme d'hépatocytes humains.

Elle a également pour but de fournir des cultures d'hépatocytes humains capables de maintenir des fonctions hépatocytaires spécifiques à un niveau élevé.

Elle vise également à fournir des cultures utilisables comme modèles pour l'étude des fonctions du foie dans des applications biologiques et biochimiques.

Le procédé de culture d'hépatocytes humains de l'invention est caractérisé en ce qu'on associe, à des hépatocytes humains en culture, des cellules d'origine hépatique, différentes des hépatocytes provenant de lignées obtenues à partir d'animaux, capables d'assurer in vivo des interactions cellulaires spécifiques avec les hépatocytes.

Dans un tel système de co-culture, il s'avère que les cellules d'origine hépatique mises en oeuvre se multiplient et viennent en confluence avec les hépatocytes, assurant ainsi l'établissement de nombreux contacts entre les deux types de cellules.

De manière inattendue, compte tenu des nombreux paramètres intervenant in vivo dans les relations intercellulaires, on constate qu'il résulte des interactions in vitro entre ces deux types de cellules, une survie prolongée des hépatocytes et un maintien à un niveau élevé de leurs fonctions spécifiques pendant cette survie.

Ces résultats constituent un avantage considérable par rapport à l'état de la technique.

On mesurera, en effet, l'intérêt d'une telle stabilité fonctionnelle à long terme, qui permet, en particulier d'apprécier les effets sur une longue période de médicaments, de carcinogènes potentiels et l'étude de diverses affections.

Les cellules d'origine hépatique sont avantageusement choisies parmi des cellules coopérant in vivo avec les hépatocytes.

Des cellules préférées de ce type sont constituées par des cellules de même nature que les hépatocytes, à savoir, des cellules épithéliales.

Des cellules d'origine hépatique provenant de lignées obtenues à partir d'animaux, sont obtenues plus spécialement à partir de petits mammifères rongeurs, notamment, compte tenu de leur disponibilité aisée, de rat.

Des cellules épithéliales de rat spécialement mises en oeuvre sont formées de cellules épithéliales telles que décrites par GUGUEN-GUILLOUZO et al. dans Exp. Cell Research 143 (1983) 47—54. Ces cellules épithéliales sont probablement d'origine biliaire.

On mesurera l'originalité de tels modèles de co-cultures de cellules provenant d'espèces différentes.

On connaît, en effet, les nombreux problèmes de spécificités d'espèces et même de spécificités tissulaires rencontrés dans les systèmes de cultures.

Or, conformément à l'invention, il s'avère que les hépatocytes humains, associés aux cellules hépatiques d'origine animale et plus spécialement, comme montré dans les exemples aux cellules hépatiques de rat, survivent au moins 6 à 8 semaines et maintiennent plusieurs fonctions différenciées telles que la secrétion d'albumine et la conjugaison de médicaments.

Selon une autre disposition de l'invention, après l'agrégation des deux types de cellules, on ajoute dans le milieu de culture mis en oeuvre une hormone plus spécialement du type corticostéroïde telle que l'hémisuccinate de l'hydrocortisone. Cette hormone a avantageusement pour effet de participer aux interactions cellulaires. Il en résulte une stabilisation des hépatocytes en culture, plus spécialement leurs fonctions spécifiques.

Selon encore une autre disposition supplémentaire de l'invention, on sépare les hépatocytes des co-cultures ainsi obtenues.

A cet effet, on soumet les co-cultures à l'action d'une enzyme protéolytique du type de la collagènase, en l'absence de son co-facteur, le calcium.

On opère avantageusement dans des conditions permettant une action lente de l'enzyme, en particulier, à une température de l'ordre de 37°C.

Alors que de nombreux travaux ont montré les difficultés de séparation des différents types cellulaires dans une culture cellulaire hétérogène, l'invention fournit donc des moyens permettant de séparer sélectivement les hépatocytes dela co-culture.

On dispose ainsi d'une population purifiée d'hépatocytes, stabilisée par la co-culture, de grand intérêt pour de nombreuses applications en biochimie et biologie moléculaire, en particulier, pour l'étude de l'expression des gènes.

Après lavage, les hépatocytes séparés des co-cultures peuvent être, soit congelés et conservés à moins 80°C environ pour utilisation ultérieure, soit réensemencés avec de nouvelles cellules hépatiques, ce qui augmente encore leur durée de vie.

Dans un mode préféré de réalisation de l'invention, on ajoute les cellules d'origine hépatique aux hépatocytes après l'attachement de ces dernièrs aux supports utilisés. Pour l'ensemencement des hépatocytes, on opère avantageusement selon les conditions habituelles, en utilisant des supports en un matériau organique tel que du polystyrène.

Les hépatocytes humains utilisés proviennent de foies adultes ou foetaux, normaux ou pathologiques, ce qui permet de disposer de plusieurs modèles pour des études en pharmacologie ou pathologie. Il est également intéressant de noter qu'avec des hépatocytes provenant de foies foetaux, on obtient au bout de quelques semaines de co-culture, une maturation du type de celle qui se produit in vivo, ce qui représente un moyen d'étude de la différenciation hépatique.

Les cellules d'origine hépatique mises en oeuvre sont avantageusement constituées comme indiqué cidessus par des cellules épithéliales de rat probablement d'origine biliaire.

Pour des études concernant le métabolisme d'un foie normal, on a plus particulièrement recours à des cellules non transformées.

Mais, on sait que ces cellules se transforment spontanément au bout de plusieurs mois. Elles modifient alors le signal donné à l'hépatocyte pour le stabiliser. L'utilisation de telles cellules transformées présentent un intérêt comme modèle pour étudier des altérations très précoces notamment en hépatocarcinogénèse.

Le milieu de culture renferme les substances nutritives utilisées généralement pour les cultures d'hépatocytes.

Il apparaît, en outre, approprié d'y incorporer de l'insuline porcine ce qui permet de favoriser l'expression des fonctions hépatocytaires.

On utilise également avantageusement de l'albumine qui a pour effet, notamment, de stabiliser les membranes ainsi que l'étalement des cellules qui s'adaptent mieux alors aux conditions de culture.

Un autre additif convenant dans ces milieux de culture est constitué par du sérum de veau.

Ce milieu est avantageusement renouvelé pendant la co-culture. En particulier, il est approprié de renouveller ce milieu, notamment tous les jours ou tous les deux jours. Ce milieu peut contenir du sérum. Mais d'une manière avantageuse, on utilise un milieu ne renfermant pas de sérum, donc mieux défini quant à sa composition et les effets qui en résultent.

Dans ce milieu de culture utilisé, après la confluence des cellules hépatiques, on ajoute un corticostéroïde avantageusement de l'hémisuccinate d'hydrocortisone, en particulier à des doses de $7 \times 10^{-5}$ M à $7 \times 10^{-7}$ M correspondant à un intervalle déterminé par la toxicité qui pourrait apparaître vis-à-vis des cultures et de la concentration physiologique.

Lorsqu'on désire isoler les hépatocytes de la co-culture, on soumet cette dernière à l'action de collagénase en l'absence de calcium.

L'absence de ce cation est connue pour entraîner des modifications dans l'adhésivité et la forme de nombreux types cellulaires.

Les conditions mises en oeuvre dans l'invention sont telles que son absence est sans effet uniquement sur les cellules épithéliales de rat.

La collagénase ayant une activité réduite agit préférentiellement sur le collagène facilement accessible, c'est-à-dire sur le collagène localisé entre les deux types cellulaires, ce qui entraîne le détachement préférentiel des groupes d'hépatocytes.

4

On réalise avantageusement cette étape en opérant une incubation de la co-culture préalablement lavée avec un tampon afin d'éliminer toute trace de calcium. On utilise environ une concentration de 0,020 à 0,030% de collagénase en solution tamponnée dépourvue de calcium et on maintient le milieu réactionnel à environ 37°C pendant 15mn.

Les hépatocytes sont récupérés en soumettant le milieu à agitation mécanique puis centrifugation et sont avantageusement lavés pour éliminer l'enzyme.

Dans un autre mode préféré de réalisation de l'invention, les cellules d'origine hépatique définies ci-dessus sont ajoutées aux populations pures d'hépatocytes après plusieurs jours de culture.

Cette disposition permet de moduler les fonctions des hépatocytes, c'est-à-dire de réactiver les gènes.

Il est ainsi possible de modifier l'activité observée avec une culture d'hépatocytes classique, où l'hépatocyte est en culture seul, par addition tardive de cellules épithéliales et de faire remonter, par exemple, la production d'albumine qui avait pratiquement complétement disparue.

La co-culture est ensuite avantageusement poursuivie dans les conditions ci-dessus.

L'invention vise également les co-cultures d'hépatocytes humains en tant que produits nouveaux.

Ces co-cultures sont caractérisées par la présence de nombreuses fibres de réticuline dès 24 heures environ après la confluence des cellules d'origine hépatique.

Elles sont également caractérisées par une stabilité fonctionnelle des hépatocytes, au regard de leurs fonctions spécifiques, d'au moins 6 à 8 semaines.

Dans un mode préféré de réalisation de l'invention, les co-cultures comprennent des hépatocytes humains en association avec des cellules hépatiques provenant de petits mammifères.

Il s'agit notamment de cellules hépatiques de petits mammifères rongeurs, en particulier, de rats.

De telles cultures sont caractérisées par la présence d'un matériau extracellulaire autour des hépatocytes et entre les hépatocytes et les cellules hépatiques.

Il s'agit d'un matériau hétérogène formé essentiellement de fibronectine et de collagènes.

Dans les co-cultures comprenant des hépatocytes provenant de foies normaux le collagène précipite et forme des fibres réticulées comme in vivo (alors qu'il reste pratiquement totalement soluble dans le milieu dans le cas de cultures pures d'hépatocytes de l'art antérieur).

Ce collagène est alors formé principalement de collagène III et comprend en moindre quantité du collagène I, ce qui traduit un bon fonctionnement des cellules.

Parallèlement, ces co-cultures sont caractérisées par le maintien à un niveau élevé de leurs fonctions hépatocytaires.

En particulier, les cellules hépatocytaires de ces co-cultures sont capables de produire de l'albumine pendant au moins quatre semaines.

La mise en évidence de cette production d'albumine par immunoperoxydase a permis de constater une polarité de l'albumine comme in vivo, c'est-à-dire une concentration des vésicules golgiennes contenant l'albumine vers un pôle de la cellule.

L'étude de la voie de glucuronidation montre par ailleurs une activité de cette dernière pendant au moins 3 semaines.

On observe, au contraire, dans le matériau extra-cellulaire une prédominance de collagène I et de faibles quantités de collagène III lorsque la secrétion d'albumine est elle-même diminuée au-delà notamment de 6 à 8 semaines de culture.

Cette évolution peut refléter le comportement connu in vivo de foies pathologiques.

Le maintien à long terme des fonctions hépatocytaires spécifiques dans les hépatocytes humains cultivés en co-culture permet d'envisager de nombreuses applications biologiques et biochimiques.

Les modèles constitués par ces co-cultures sont utilisables, notamment en cancérologie pour des tests de mutagénése et de cancérogénése; en pharmacologie pour des tests d'hépatotoxicité aigüe ou chronique des médicaments, de biotransformation hépatique d'une molécule nouvelle (au cours de la période de recherche et de développement d'un principe pharmacologiquement actif), également en biotechnologie pour la production de métabolites de médicaments, de protéines plasmatiques humaines (en particulier l'albumine et les facteurs de coagulation), de facteurs de suppléance hépatique, utilisables en cas de grande insuffisance hépatique cellulaire, production d'antigènes viraux (en particulier ceux du virus de l'hépatite B).

D'autres caractéristiques et avantages de l'invention apparaîtront dans les exemples qui suivent et en se reportant aux figures 1 à 6.

La figure 1a représente une photo au microscope optique (grossissement 120) montrant le matériau extracellulaire après coloration de réticuline (a), et les figures 1b et 1c, les photos obtenues par immunofluorescences après 22 jours de culture, montrant respectivement la fibronectine (1b) et le collagène III (1c),

les figures 2a et 2b représentent des photos prises au microscope à contraste de phase, respectivement de cultures d'hépatocytes seuls et de co-cultures selon l'invention,

— la figure 3 représente une photo prise au microscope électronique de co-cultures de l'invention montrant des fibres de collagène entre deux types cellulaires,

— la figure 4 représente une courbe de la variation de la concentration d'albumine en fonction du temps avec des cultures conventionnelles d'une part et avec des co-cultures de l'invention d'autre part,

— la figure 5 représente les formules du kétotifène et de ses métabolites, et,

— les figures *6a* à *6c* représentent des photos au microscope montrant respectivement des celles d'hépatocytes se détachant d'une co-culture, des hépatocytes isolés et les plages laissées libres dans la co-culture après séparation des hépatocytes.

Exemple 1: Procédé de co-culture d'hépatocytes humains et de cellules hépatiques de rat.

On rapporte sous a) les moyens utilisés pour prélever les hépatocytes humains et sous b) les conditions mises en oeuvre pour la co-culture.

a) prélèvement des hépatocytes.

On opère au terme de binephrectomie pour transplantation rénale selon la technique décrite par GUGUEN-GUILLOUZO et al dans Cell. Biol. Int. Rep. 1982, 6, 625—628 "High yield preparation of isolated human adult hepatocytes by enzymatic perfusion of the liver"

Les hépatocytes humains sont obtenus à partir soit de fragments de tissu hépatique (5—10 g) soit d'une partie du foie entier (lobe gauche). Le tissu est dissocié par perfusion d'une solution de collagénase via la veine porte. Les hépatocytes fraîchement isolés présentent les caractéristiques d'ultrastructure des cellules du parenchyme in vivo. Le procédé d'isolement ci-dessus n'entraîne aucune altération nucléaire ou cytoplasmique évidente.

Le rendement cellulaire est de l'ordre de $10^9$ hépatocytes et la viabilité des cellules de l'ordre de 85%.

Les hépatocytes isolés sont lavés et purifiés avant leur mise en culture.

b) co-culture-

Les hépatocytes sont alors ensemencés dans des flacons de polystyrène comportant ou non un revêtement de fibronectine purifiée à partir de plasma humain.

L'ensemencement est réalisé à raison de $0,5 \times 10^6$ cellules par ml de milieu de culture.

On utilise un milieu $HamF_{12}$ contenant 10 µg/ml d'insuline porcine, 0,2% d'albumine bovine et 10% de sérum de veau foetal. Environ 3 heures plus tard, le temps nécessaire pour l'attachement de 60 à 70% des hépatocytes au substrat, le milieu nutritif est enlevé et remplacé par un milieu nutritif identique frais contenant $0,8 \times 10^6$ cellules épithéliales hépatiques de rat par ml. Ces cellules proviennent de lignées obtenues par dissociation à l'aide d'une solution de trypsine de foie de rat âgé de 10 jours. Elles sont utilisées avant leur transformation spontanée in vitro, facilement reconnaissable selon divers critères comme rapporté par MOREL-CHANY E. et al dans "Spontaneous neoplastic transformation in vitro of epithelial cell strains of rat liver: cytology, growth and enzymatic activities." Europ.3. Cancer, 1978, *14* 1341—1352.

Au cours des heures suivantes, les hépatocytes s'étalent pour constituer des travées de cellules épithéliales granulaires. Les cellules épithéliales hépatiques de rat prolifèrent et occupent rapidement tous les espaces libres.

Après 24—30 h. une confluence cellulaire est atteinte. Le milieu nutritif est renouvelé tous les jours ou tous les 2 jours. Lorsque la confluence cellulaire est atteinte, on ajoute dans ce milieu $3,6 \times 10^{-5}$ M d'hémisuccinate d'hydrocortisone. A partir de ce moment la présence de sérum n'est pas nécessaire.

Dans ces conditions, les hépatocytes survivent sans perte cellulaire évidente pendant au moins 6 à 8 semaine's avec maintien à un niveau élevé de diverses fonctions spécifiques. On n'observe ni prolifération évidente d'hépatocytes ou de cellules épithéliales de rat, ni détachement d'un nombre significatif d'hépatocytes.

On remarque, par étude au microscope électronique, la production d'un materiel extracellulaire important.

Ce matériel comporte de nombreuses fibres qui apparaissent dès 24 h. après la confluence cellulaire et sont localisées entre les deux populations cellulaires et entre les hépatocytes.

On note également la présence en quantité importante de fibronectine et de collagène de type III. En revanche, on n'observe que de faibles quantités de collagène de type I autour des hépatocytes. On trouve également du collagène de type IV et de la laminine.

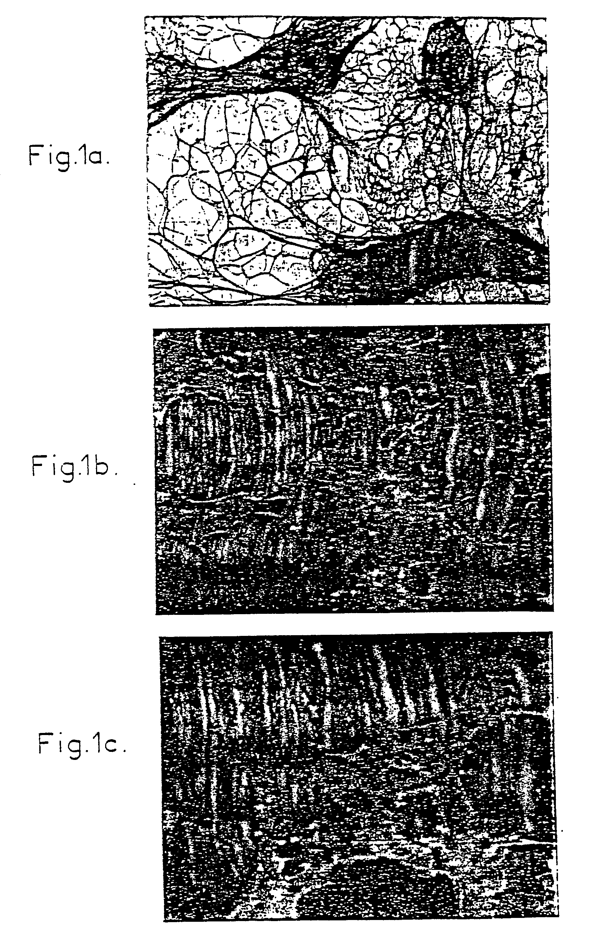

La figure 1 correspond à une photo prise au microscope optique (grossissement 120) sur laquelle apparaît le matériau extracellulaire en question après coloration de réticuline au bout de 6 jours de culture (a) et où sont localisés de la fibronectine (b) et du collagène de type III (c) par immunofluorescence indirecte après 22 jours de culture.

Exemple Comparatif IA: culture d'hépatocytes humains selon une technique conventionnelle

On recouvre des flacons de polystyrène comportant ou non un revêtement de fibronectine purifiée à partir de plasma humain à l'aide d'hépatocytes prélevés comme indiqué dans l'exemple 1.

L'ensemencement est effectué à raison de $2 \times 10^6$ cellules par flacon dans 3 ml du milieu de culture utilisé également pour l'ensemencement ci-dessus mais dans lequel on ajoute $5 \times 10^{-5}$ M d'hémisuccinate d'hydrocortisone. Ce milieu est renouvellé 4 h après l'ensemencement, puis chaque jour.

L'étude au microscope électronique ne revèle que peu de fibres réticulaires entre les cellules.

Exemple 2: Comparaison entre les caractéristiques morphologiques et les activités fonctionnelles des hepatocytes en co-culture (selon l'exemple 1) ou en culture conventionnelle (selon l'exemple comparatif 1A)

*caractéristiques morphologiques*

En utilisant les conditions de culture habituelle, on observe un étalement rapide des hépatocytes, en particulier lorsqu'on utilise des flacons recouverts de fibronectine.

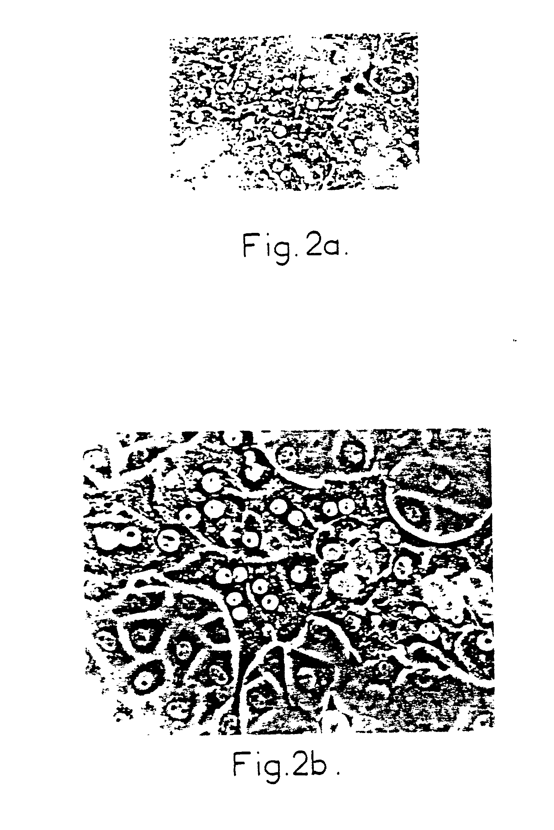

Comme le montre la figure 2a, il se forme des mono-couches de cellules épithéliales granulaires avec des espaces intercellulaires réfringents correspondant à des structures du type des canalicules biliaires. Cette figure correspond à une photo prise après 4 jours de culture, à un grossissement de 290 avec un microscope à contraste de phase. La survie de ces cellules n'excède pas 2 à 3 semaines. L'examen de ces cellules au niveau de leur ultrastructure montre, après quelques jours, une diminution progressive des particules de glycogène et du réticulum endoplasmique granulaire accompagnée d'une augmentation rapide des éléments du cytosquelette en particulier sous la membrane plasmatique.

Au contraire, comme le montre l'examen de la figure 2b, les hépatocytes obtenus selon le procédé de co-culture de l'invention sont moins étalés que dans le cas des cultures classiques. Cette figure 2b correspond à une photo de cultures âgées de 10 jours prise au microscope à contraste de phase (grossissement 200).

L'examen de cette photo permet d'observerdes structures du type canalicules biliaires. On remarque que les particules de glycogène restent abondantes et les complexes GOLGI sont localisés près des structures du type des canaux biliaires.

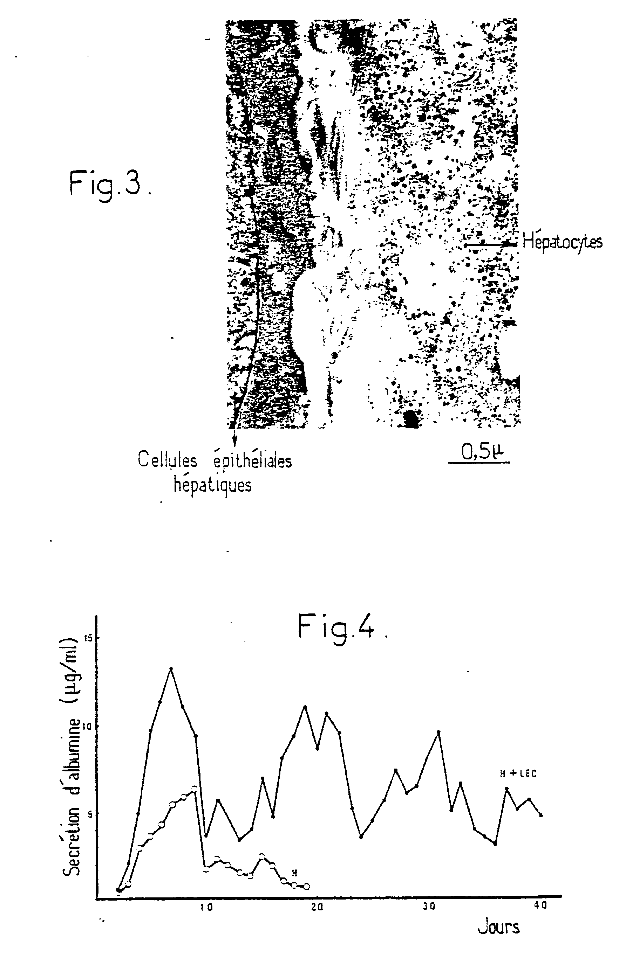

La photo représentée sur la figure 3, prise au microscope électronique après 30 jours de culture (grossissement 31 000) permet d'observer de nombreux contacts entre les deux populations cellulaires et des structures filamenteuses comprenant des fibres typiques de collagène apparaissant dans les espaces intracellulaires (voir flèche).

*Activités fonctionnelles: secrétion d'albumine.*

On a étudié la secrétion d'albumine en fonction du temps avec des hépatocytes de cultures classiques (H) et des hépatocytes en coculture (HC), en milieu sans sérum.

Sur la figure 4, on a rapporté les résultats respectivement obtenus correspondant à la moyenne de deux expériences, Les courbes ●——● et ○——○ correspondent respectivement à l'utilisation de HC et de H en l'absence de sérum de veau.

Avec des conditions de culture habituelle, on constate une augmentation de la secrétion d'albumine en fonction du temps durant les 8 premiers jours, suivie d'une diminution.

Dans le cas des co-cultures, la vitesse de secrétion d'albumine s'avère plus élevée et reste à un niveau élevé pendant plusieurs semaines, même en l'absence de sérum. En utilisant la technique de l'immunoperoxydase, on met en évidence une production d'albumine par la plupart des hépatocytes durant au moins les quatre première semaines de culture.

*modulation de la production d'albumine*

On utilise des cultures d'hépatocytes humains seuls en présence ou en l'absence de sérum de veau foetal.

Au bout de quatre jours de cultures, la production d'albumine est très faible, voire pratiquement nulle.

En ajoutant des cellules épithéliales hépatiques de rat conformément à l'invention, on note une reprise de la production d'albumine qui atteint alors un niveau élevé.

L'utilisation de co-cultures de l'invention permet donc de réactiver les fonctions spécifiques des hépatocytes.

*biotransformation du kétotifène*

Le kétotifène commercialisé sous la marque ZADITEN par les laboratoires SANDOZ est un médicament administré par voie orale à action anaphylactique.

Ce médicament a été choisi pour cette étude en raison de sa métabolisation selon différentes voies typiques comme décrit par M. GUERRET et al dans Proceedings 1 st Eur. Cong. Biopharm. Clermont-Ferrand, Vol. I, p 317, Techn. et Doc. (1981).

L'étude de cette biotransformation (qui sera rapportée plus en détail dans l'exemple 3 suivant) montre que la voie de glucuronidation reste le plus active pendant au moins 3 semaines avec des hépatocytes en co-cultures alors qu'elle disparaît après 4 jours avec des cultures d'hépatocytes conventionnelles.

Exemple 3: application des cultures d'hépatocytes humains adultes comme modèle pour l'étude du métabolisme de médicaments

Chez l'homme, le kétotifène est biotransformé selon plusieurs voies métaboliques, subissant une N-déméthylation, une N-oxydation, une N-glucuroconjugaison conduisant à un dérivé d'ammonium quaternaire et à une réduction du groupe céto suivie d'une O-glucuronidation. Cette biotransformation est décrite dans l'article de GUERET et al cité plus haut.

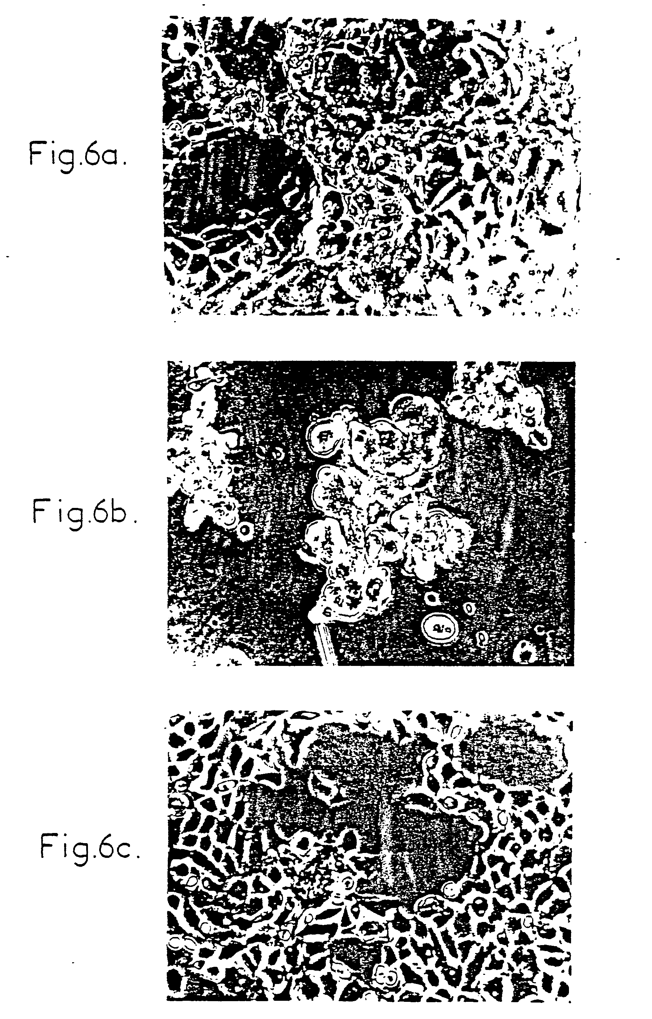

Sur la figure 6, on a rapporté les formules du kétotifène et des métabolites obtenus. Ces formules portent les N° 1 à 6 et correspondent respectivement: 1) au kétotifène 2) au kétotifène réduit, 3) au N-glucurokétotifène, 4) au nor-kétotifène, 5) au nor-kétotifène réduit et 6) au kétotifène oxydé.

A différents intervalles de temps après l'ensemencement des cellules, entre 24 h et 3 semaines, on incube les hépatocytes adultes d'une culture réalisée selon l'exemple 1 avec 3 µg/ml de kétotifène -$^{14}$C

7

ayant une activité spécifique de 107μCi/mg et 20 μg/ml de médicament non marqué par 25 cm² de flacon.

On ne note aucune toxicité à cette concentration en médicament de 23 μg/ml de milieu.

Quatre heures ou 24 heures après l'addition de médicament, on recueille des quantités aliquotes de milieu qu'on congèle rapidement et qu'on stocke à -20°C jusqu'à analyse. Les métabolites du kétotifène sont analysés par chromatographie en phase liquide à haute performance (HPLC) à phase inversée. Leur structure est confirmée par spectrométrie de masse.

La détection limite de radioactivité est de 400 dpm ce qui correspond à 0,13 ng de¹⁴C-kétotifène.

Les temps de rétention des métabolites qui sont semblables dans les deux expériences sont comparés avec ceux obtenus avec des composés de synthèse.

On recueille les métabolites aux fins d'étude par spectrométrie de masse.

De même, on analyse des médicaments analogues et leurs métabolites avant et après incubation de β-glucuronidase.

Les expériences réalisées ont montré que quelque soit le temps de culture, on ne détecte pas de glucuronides du kétotifène avant 8 heures suivant l'addition de médicament.

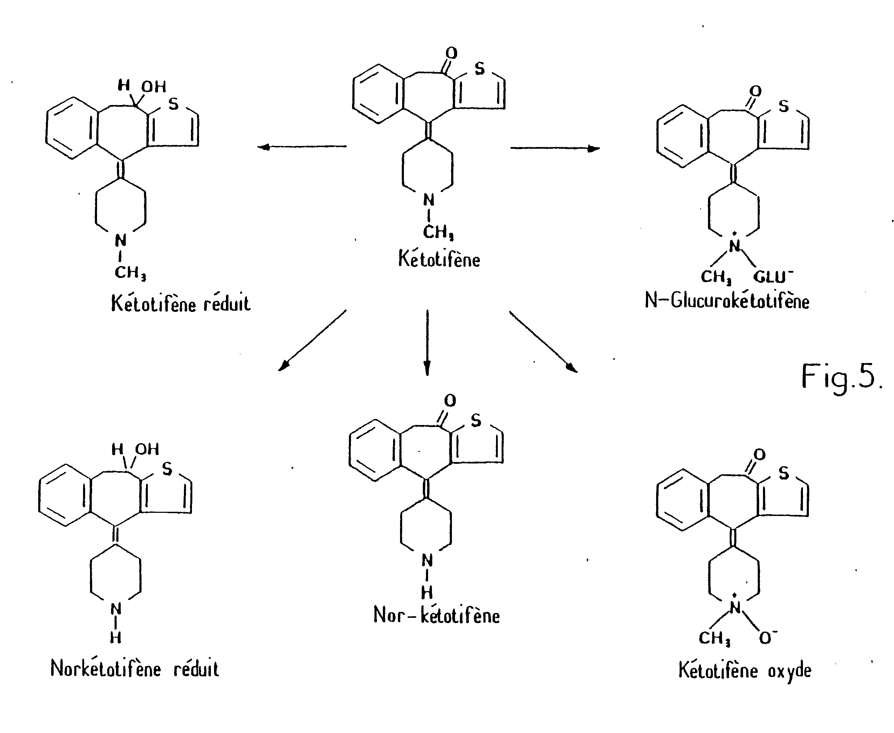

Entre 4 et 28 h après l'ensemencement des cellules incubées avec le kétotifène, on obtient, comme représenté sur la figure 5, les métabolites suivant dans le milieu de culture, à savoir

— du nor-kétotifène: ce produit possède le temps de rétention le plus long en HP C (74 mn) et donne union moléculaire caractéristique à m/e 295 en GLC-MS;

— des traces de kétotifène N-oxyde qui se décompose à l'analyse GLC-MS donnant le médicament non modifié,

— du kétotifène réduit constituant le principal métabolite: ce produit possède en HPLC un temps de rétention identique à celui du composé de synthèse de référence obtenu par réduction de kétotifène avec du borohydrure de sodium. A l'analyse GLC-MC, les ions moléculaires et autres caractéristiques ioniques sont observés à m/e 311 et m/e 291 respectivement (M⁺—18(H₂O)).

— du nor-kétotifène réduit donnant en GLC-MS un ion de fragment caractéristiqueà m/e 279 (M⁺—18 (H₂O));

— des glucuronides à la fois du médicament non modifié (N-glucuro-kétotifène) et de kétotifène réduit, obtenus également en quantités importantes entre 20 et 25 mn en HPLC.

Ces produits conduisent à du kétotifène et à son dérivé réduit libre après incubation avec de la β-glucuronidase. On trouve également de faibles quantités de glucuronide de nor-kétotifène réduit.

Ces différents métobolites correspondent aux quatre voies métaboliques trouvées in vivo, à savoir la N-déméthylation, la N-oxydation et principalement la cétoréduction et la N-glucuronidation.

Ces deux dernières voies sont utilisées afin de déterminer l'activité métabolique de cellules cultivées seules ou enassociation avec des cellules épithéliales de foie durant 24 h. ou plus.

Après incubation de 4 h. de kétotifène et d'hépatocytes humains de culture, la vitesse du métabolisme apparaît faiblement modifiée les 7 premiers jours dans les deux cas. Avec des hépatocytes de co-cultures, la métabolisation du kétotifène s'effectue de manière plus active.

| Cellules | Durée de Culture (jours) | Ketotifene non modifié | | Ketotifene Metabolise | | | | Rapport G / R G |
|---|---|---|---|---|---|---|---|---|
| | | | | Reduit (R) | | N-Glucuronide (G) | | |
| | | a | b | a | b | a | b | b |
| Hepatocytes | 0.17 | 67 | 25 | 33 | 48 | 0 | 25 | 0.34 |
| " | 1 | 68 | 16 | 32 | 56 | 0 | 28 | 0.33 |
| " | 4 | 63 | 13 | 37 | 61 | 0 | 26 | 0.30 |
| " | 6 | 93 | 50 | 7 | 50 | 0 | 0 | 0.00 |
| Hepatocytes + cellules épithéliales de foie | 4 | 58 | 13 | 42 | 43 | 0 | 44 | 0.51 |
| " | 7 | 51 | 6 | 49 | 29 | 0 | 65 | 0.69 |
| " | 21 | 83 | 29 | 17 | 59 | 0 | 12 | 0.17 |
| Cellules épithéliales de foie | | 100 | 100 | 0 | 0 | 0 | 0 | — |

La vitesse du métabolisme se réduit considérablement avec des cultures plus âgées. Elle apparaît cependant beaucoup plus élevée au 21ème jour de co-culture qu'au 6ème jour de culture conventionnelle.

Le tableau ci-dessous comporte les résultats relatifs aux pourcentages respectifs de kétotifène non modifié, de kétotifène réduit et de kétotifène de N-glucuronide obtenus 4 h et 24 h après l'addition de médicament.

Les valeurs indiquées correspondent à la moyenne de deux essais.

Comme le montre l'examen de ce tableau, avec les cultures conventionnelles, le rapport du nombre de métabolite glucuronide au nombre de métabolites totaux (métabolites réduits et N-glucuronide) est de 0,34 à 0,30 les 4 premiers jours.

Le 6ème jour. on ne note plus d'activité de glucuronidation alors qu'on observe encore une réduction.

Avec les co-cultures, le kétotifène est activement métabolisé durant la période étudiée de 21 jours.

Au 7ème jour, le rapport métabolite de glucuronide: métabolites totaux est de 0.69 et au 21ème jour, ce rapport est encore de 0,17.

A ce moment là, le pourcentage de réduction de kétotifène total est identique à celui trouvé dans des cultures conventionnelles après 24 h.

Durant les 21 jours de co-culture on détecte du nor-kétotifène.

Dans les cultures de cellules épithéliales utilisées comme contrôle, on ne détecte aucune biotransformation en kétotifène.

Exemple 4: Production d'antigènes viraux

Des cultures d'hépatocytes humains normaux ont été infestées expérimentalement par le virus B de l'hépatite.

Une fois infectés, on a montré la production des antigènes viraux $Hb_s$ et $Hb_e$ caractéristiques du virus.

L'étude réalisée a également montré le maintien à long terme, à taux plus élevé que dans les cultures infestées de l'art antérieur, de la production de ces antigènes pendant au moins trois semaines. La production maintenue de $Hb_e$ montre une réplication du virus.

Les co-cultures d'hépatocytes humains de l'invention fournissent donc des modèles précieux notamment pour l'étude des facteurs impliqués dans la reconnaissance par le virus de la cellule cible.

Selon un autre aspect, elles constituent également des moyens de grand intérêt pour l'obtention d'antigènes viraux et d'anticorps spécifiques en quantité élevée.

Exemple 5: Séparation des hépatocytes d'une co-culture d'hépatocytes humains et de cellules épithéliales de rat

On soumet une co-culture du type de l'exemple 1 à deux lavages successifs afin d'éliminer toute trace de calcium du milieu de culture. On utilise une solution saline du type PBS (tampon phosphate borate sodique), dépourvue de calcium et de magnésium.

On opère une incubation à 37°C pendant 15mn de la co-culture avec une solution aqueuse de collagénase préparée en concentration de 0,025 % dans une solution tampon elle-même dépourvue de calcium.

On soumet la co-culture à une agitation mécanique pour décoller les hépatocytes. On recueille ceux-ci par centrifugation, puis on les met en suspension et on les lave pour éliminer la collagénase.

Les hépatocytes isolés sont immédiatement utilisés pour de nouvelles cultures ou congelés pour conservation.

Sur les figures *6a* à *6c*, on a représenté respectivement les populations d'hépatocytes se détachant sous l'action de la collagénase de la co-culture (*6a*), les populations séparées (6b) et la culture restante avec les plages laissées libres par l'enlèvement des hépatocytes (*6c*).

**Revendications**

1. Procédé de culture d'hépatocytes humains, caractérisé en ce qu'on associe, à des hépatocytes humains en culture, des cellules d'origine hépatique, provenant de lignées obtenues à partir d'animaux, différentes des hépatocytes, capables d'assurer in vivo des interactions cellulaires spécifiques avec les hépatocytes.

2. Procédé selon la revendication 1, caractérisé en ce que les cellules d'origine hépatique sont des cellules hépatiques épithéliales.

3. Procédé selon la revendication 1 ou 2, caractérisé en ce que les lignées de cellules d'origine animale sont obtenues à partir de petits mammifères rongeurs, notamment, à partir de rats.

4. Procédé selon l'une quelconque des revendications 1 à 3, caractérisé en ce qu'on ajoute dans le milieu de culture une hormone de type corticostéroïde, telle que l'hémisuccinate d'hydrocortisone, avantageusement après agrégation des deux types de cellules.

5. Procédé selon l'une quelconque des revendications précédentes, caractérisé en ce que, pendant la co-culture, on utilise un milieu de culture renfermant de $7 \times 10^{-5}$M à $7 \times 10 \times {}^{-7}$M environ d'hémisuccinate d'hydrocortisone.

6. Procédé selon l'une quelconque des revendications précédentes, caractérisé en ce qu'on utilise pendant la co-culture un milieu sans sérum.

EP 0 143 809 B1

7. Procédé selon l'une quelconque des revendications 1 à 6, caractérisé en ce que le milieu de culture renferme de l'insuline porcine ainsi qu'avantageusement de l'albumine bovine et, lors de l'ensemencement des hépatocytes du sérum tel que du sérum foetal de veau.

8. Procédé selon l'une quelconque des revendications précédentes, caractérisé par l'étape supplémentaire de séparation des hépatocytes de la co-culture par action d'une enzyme protéolytique du type de la collagènase dans un milieu dépourvu de calcium, dans des conditions permettant une action lente de la collagénase, spécifique vis-à-vis des populations d'hépatocytes, puis qu'on soumet la co-culture à un traitement mécanique pour récupérer les hépatocytes.

9. Procédé selon la revendication 8, caractérisé en ce qu'on opère l'incubation de la co-culture avec la collagénase à environ 37°C pendant environ 15mn, à une concentration d'environ 0,020 à 0,030% en solution tamponnée dépourvue de calcium.

10. Procédé selon l'une quelconque des revendications 1 à 9, caractérisé en ce que les cellules d'origine hépatique sont ajoutées à la culture d'hépatocytes dès l'agrégation de ces derniers.

11. Procédé selon l'une quelconque des revendications 1 à 9, caractérisé en ce que les cellules d'origine hépatique sont ajoutées aux populations pures d'hépatocytes après plusieurs jours de culture, ce qui permet de moduler les fonctions des hépatocytes.

12. Procédé selon l'une quelconque des revendications 1 à 11, caractérisé en ce que les hépatocytes humains proviennent de foies normaux ou pathologiques, adultes ou foetaux.

13. Procédé selon l'une quelconque des revendications précédentes, caractérisé en ce que les cellules épithéliales d'origine hépatique sont des cellules transformées ou non transformées.

14. Co-cultures d'hépatocytes humains, caractérisées en ce qu'elles comprennent en association des hépatocytes humains et des cellules d'origine hépatique tel que défini dans l'une quelconque des revendications 1 à 4, 12 ou 13.

15. Co-cultures selon la revendication 14, caractérisées par la présence de nombreuses fibres de réticuline dès 24 heures environ après la confluence des cellules d'origine hépatique.

16. Co-cultures selon la revendication 14 ou 15, caractérisées par une stabilité fonctionnelle des hépatocytes, au regard de leurs fonctions spécifiques, d'au moins 6 à 8 semaines.

17. Co-cultures selon l'une quelconque des revendications 14 à 16, caractérisées en ce que les cellules d'origine hépatique sont des cellules épithéliales de petits mammifères rongeurs, en particulier de rats et que les co-cultures résultantes comportent un matériau extracellulaire autour des hépatocytes et entre les hépatocytes et les cellules hépatiques, ce matériau étant hétérogène et formé essentiellement de fibronectine et de collagène.

18. Co-cultures selon la revendication 17, caractérisées en ce que, dans les co-cultures comprenant des hépatocytes provenant de foies normaux, le collagène est formé principalement de collagène III et comprend en moindre quantité du collagène I, et que parallèlement les fonctions hépatocytaires sont maintenues à un niveau élevé et que lorsque la secrétion d'albumine est elle-même diminuée, le matériau extra-cellulaire comporte une majeure partie de collagène I et de faible quantités de collagène III.

19. Utilisations biologiques et biochimiques des co-cultures selon l'une quelconque des revendications 14 à 18, et des hépatocytes séparés de ces co-cultures, en particulier comme modèles pour l'étude des fonctions hépatocytaires en pharmacologie et en pathologie.

**Patentansprüche**

1. Verfahren zum Züchten von Human-Hepatozyten, dadurch gekennzeichnet, daß man mit in einer Kultur vorliegenden Human-Hepatozyten Zellen heptischen Ursprungs vereinigt, welche Zellen aus Zellinien stammen, die aus Tieren erhalten worden sind, und die von Hepatozyten verschieden sowie befähigt sind, in vivo spezifische zellulare Wechselwirkungen mit den Hepatozyten zu gewährleisten.

2. Verfahren nach Anspruch 1, dadurch gekennzeichnet, daß die Zellen hepatischen Ursprungs Leberepithelzellen sind.

3. Verfahren nach Anspruch 1 oder 2, dadurch gekennzeichnet, daß die aus Tieren stammenden Zellinien aus kleinen Nagersäugetieren, insbesondere aus Ratten gewonnen werden.

4. Verfahren nach einem der Ansprüche 1 bis 3, dadurch gekennzeichnet, daß mam zu dem Kulturmedium ein Hormon vom Typus der Corticosteroide, wie z,B, das Hydrocortisonhemisuccinat, hinzufügt, und zwar vorteilhafterweise nach der Aggregation der beiden Zelltypen.

5. Verfahren nach einem der vorhergehenden Ansprüche, dadurch gekennzeichnet, daß man während der Ko-Kultur ein Kulturmedium verwendet, welches etwa $7 \times 10^{-5}$M bis $7 \times 10^{-7}$M Hydrocortisonhemisuccinat enthält.

6. Verfahren nach einem der vorhergehenden Ansprüche, dadurch gekennzeichnet, daß man während der Ko-Kultur ein Kulturmedium ohne Serum verwendet.

7. Verfahren nach einem der Ansprüche 1 bis 6, dadurch gekennzeichnet, daß das Kulturmedium Schweineinsulin sowie vorteilhafterweise Rinderserumalbumin und, zum Zeitpunkt des Beimpfens der Hepatozyten, ein Serum, wie z.B. Kälberfetalserum, enthält.

8. Verfahren nach einem der vorhergehenden Ansprüche, gekennzeichnet durch die ergänzende Stufe einer Abtrennung der Hepatozyten von der Ko-Kultur durch Einwirkung eines proteolytischen Enzyms vom Typus der Kollagenase in einem calciumfreien Medium, unter Bedingungen, welche ein largsames,

11

spezifisches Einwirken der Kollagenase auf die Hepatozytenpopulationen ermöglichen, worauf man die Ko-Kultur einer mechanischen Behandlung zu Gewinnung der Hepatozyten unterwirft.

9. Verfahen nach Anspruch 8, dadurch gekennzeichnet, daß man die Bebrütung der Ko-Kultur mit der Kollagenase bei etwa 37° C und während etwa 15 Minuten bei einer Konzentration von etwa 0,020 bis 0,030 % in calciumfreier, gepufferter Lösung durchführt.

10. Verfahren nach einem der Ansprüche 1 bis 9, dadurch gekennzeichnet, daß die Zellen hepatischen Ursprungs zu der Hepatozytenkultur zugegeben werden, sobald eine Aggregation der Hepatozyten stattgefunden hat.

11. Verfahren nach einem der Ansprüche 1 bis 9, dadurch gekennzeichnet, daß die Zellen hepatischen Ursprngs zu den reinen Hepatozytenkulturen nach mehreren Tagen des Züchtens zugesetzt werden, was ein Modulieren der Hepatozytenfunktionen ermöglicht.

12. Verfahren nach einem der Ansprüche 1 bis 11, dadurch gekennzeichnet, daß die Human-Hepatozyten aus mormalen oder pathologischen Erwachsenen- oder Fetallebern stammen.

13. Verfahren nach einem der vorhergehenden Ansprüche, dadurch gekennzeichnet, daß die Epithelzellen hepatischen Ursprngs transformierte oder nicht transformierte Zellen sind.

14. Ko-Kulturen von Human-Hepatozyten, dadurch gekennzeichnet, daß sie eine Vereinigung von Human-Hepatozyten und von Zellen hepatischen Ursprungs umfassen, wie dies in einem der Ansprüche 1 bis 4, 12 oder 13 definiert ist.

15. Ko-Kulturen nach Anspruch 14, gekennzeichnet durch das Vorliegen von zahlreichen Retikulinfasern ab einem Zeitpunkt von etwa 24 Stunden nach dem Konfluieren der Zellen hepatischen Ursprungs.

16. Ko-Kulturen nach Anspruch 14 oder 15, gekennzeichnet durch eine funktionelle Stabilität der Hepatozyten, in bezug auf ihre spezifischen Fumktionen, von wenigstens 6 bis 8 Wochen.

17. Ko-Kulturen nach einem der Ansprüche 14 bis 16, dadurch gekennzeichnet, daß die Zellen hepatischen Ursprungs Epithelzellen aus kleinen Nagersäugetieren, insbesondere Ratten, sind, und daß die entstandenen Ko-Kulturen rund um die Hepatozyten herum und zwischen den Hepatozyten und den hepatischen Zellen ein extrazellulares Material aufweisen, wobei dieses Material heterogen ist und im wesentlichen aus Fibronectin umd Kollagen gebildet ist.

18. Ko-Kulturen nach Anspruch 17, dadurch gekennzeichnet, daß die Ko-Kulturen Hepatozyten enthalten, die aus normalen Lebern stammen, daß das Kollagen hauptsächlich aus Kollagen III aufgebaut ist und eine geringe Menge an Kollagen I enthält, un daß gliechlaufend dazu die Hepatozytenfunktionen auf einem hohen Niveau gehalten werden, und daß dann, wenn die Albuminsekretion als solche vermindert ist, das extrazellulare Material einen Hauptanteil an Kollagen I und geringe Mengen an Kollagen III enthält.

19. Biologische und biochemische Anwendungen der Ko-Kulturen nach einem der Ansprüche 14 bis 18, und der von diesen Ko-Kulturen abgetrennten Hepatozyten, und zwar insbesondere als Modelle für das Studium von Hepatozytenfunktionen in der Pharmakologie und in der Pathologie.

## Claims

1. Culture method for human hepatocytes, characterized in that there are associated, with a culture of human hepatocytes, cells of hepatic origin, different from the hepatocytes, originating from lines obtained from animals ensuring in vivo specific cellular interactions with the hepatocytes.

2. Method according to claim 1, characterized in that the cells of hepatic origin are epithelial hepatic cells.

3. Method according to claim 1 or 2, characterized in that the cell lines of animal origin are obtained from small rodent mammals, particularly, from rats.

4. Method according to anyone of claims 1 or 3, characterized in that there is added into the culture medium a hormone of the corticosteroid type, such as hydrocortisone hemisuccinate, advantageously after agregation of the two types of cells.

5. Method according to anyone of the preceding claims, characterized in that during the co-culture a culture medium is used containing from $7 \times 10^{-5}$M to $7 \times 10^{-7}$M approximately of hydrocortisone hemisuccinate.

6. Method according to anyone of the preceding claims, characterized in that during the co-culture a serum-free medium is used.

7. Method according to anyone of claims 1 to 6, characterized in that the culture medium contains pig insulin as well as advantageously bovine albumin and during the seeding of the hepatocytes serum such as fetal calf serum.

8. Method according to anyone of the preceding claims, characterized by the additional step of separation of the hepatocytes from the co-culture by the action of a proteolytic enzyme of the type of collagenase in a medium devoid of calcium, under conditions enabling a slow action of the collagenase, specific with respect to the hepatocyte populations, and then subjecting the co-culture to mechanical treatment to recover the hepatocyte.

9. Method according to claim 8, characterized in that the incubation of the co-culture is performed with collagenase at about 37°C for about 15 min., at a concentration of about 0.020 to 0.030% in buffered solution devoid of calcium.

10. Method according to anyone of claims 1 to 9, characterized in that the cells of hepatic origin are added to the hepatocyte culture on the agregation of the latter.

11. Method according to anyone of claims 1 to 9, characterized in that the cells of hepatic origin are added to pure hepatocyte populations after several days of culture, which permits the functions of the hepatocytes to be modulated.

12. Method according to anyone of claims 1 to 11, characterized in that the human hepatocytes are obtained from adult or fetal, normal or pathological livers.

13. Method according to anyone of the preceding claims, characterized in that the epithelial cells of hepatic origin are transformed or untransformed cells.

14. Human hepatocyte co-cultures, characterized in that they comprise in association human hepatocytes and cells of hepatic origin as defined in any one of claims 1 to 4, 12 or 13.

15. Co-cultures according to claim 14, characterized by the presence of numerous reticulin fibres from about 24 hours after the confluence of the cells of hepatic origin.

16. Co-cultures according to claim 14 or 15, characterized by a functional stability of the hepatocytes, with regard to their specific functions, of at least 6 to 8 weeks.

17. Co-cultures according to any one of claims 14 to 16, characterized in that the cells of hepatic origin are epithelial cells of small rodent mammals, in particular rats and that the resulting co-cultures include an extracellular material around the hepatocytes and between the hepatocytes and the hepatic cells, this material being heterogeneous and formed essentially from fibronectin and collagen.

18. Co-cultures according to claim 17, characterized in that the co-cultures comprising hepatocytes coming from normal livers, collagen is formed principally of collagen III and comprises in the least amount collagen I, and in parallel the hepatocyte functions are maintained at a high level and that when the albumin secretion is itself diminished, the extracellular material comprises a major part of collagen I and small amounts of collagen III.

19. Biological and biochemical uses of the co-cultures according to any one of claims 14 to 18, and of the hepatocytes separated from these co-cultures, in particular as models for the study of hepatocyte functions in pharmacology and in pathology.

Fig.1a.

Fig.1b.

Fig.1c.

Fig. 2a.

Fig. 2b.

Fig.3.

Hépatocytes

Cellules épithéliales
hépatiques

0,5μ

Fig.4.

H + LEC

H

Jours

3

Fig. 5.

N-Glucurokétotifène

Kétotifène oxyde

Kétotifène

Nor-kétotifène

Kétotifène réduit

Norkétotifène réduit

Fig.6a.

Fig.6b.

Fig.6c.